# EUROPÄISCHE PATENTANMELDUNG

(11) **EP 0 847 990 A1**
(43) Veröffentlichungstag der Anmeldung: **17.06.1998**
(21) Anmeldenummer: 96119901.5
(22) Anmeldetag: 21.10.1991
(51) Int. Cl.: C07D 207/38, C07D 207/273

(54) **4-(C1-C4)-Alkoxy-3-pyrrolin-2-on-1-yl-essigsäuren, deren Herstellung und Verwendung zur Synthese von 4-Hydroxy-2-oxo-pyrrolidin-1-yl-acetamid**

(62) Teilanmeldung aus: 91117929.9
(71) Anmelder: LONZA AG, CH-3945 Gampel/Wallis (CH)
(72) Erfinder: Laffan, David, Dr., London SW5 9DL (GB); Bänziger, Markus, Dr., 3902 Brig-Glis (CH); McGarrity, John, Dr., 3902 Brig-Glis (CH)
(74) Vertreter: Weinhold, Peter, Dr.

(57) **Zusammenfassung**

Es werden neue 4-(C₁-C₄)-Alkoxy-3-pyrrolin-2-on-1-yl-essigsäuren der allgemeinen Formel II 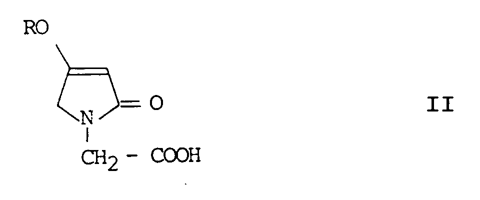 beschrieben, worin R (C₁-C₄)-Niederalkyl, vorzugsweise Methyl oder Ethyl, bedeutet.

Diese Verbindungen werden aus 4-Halogen-3-(C₁-C₄)-alkoxy-2E-butensäure-(C₁-C₄)-alkylestern durch Umsetzung mit Glycin hergestellt.

Sie eignen sich zur Herstellung des 4-Hydroxy-2-oxo-pyrrolidin-1-yl-acetamids, einem cerebral wirksamen Pharmazeutikum. Hierzu werden die Alkylgruppen sauer hydrolysiert, die resultierende 2,4-Dioxopyrrolidin-1-yl-essigsäure in Gegenwart eines Hydrierkatalysators mit Wasserstoff hydriert, die gebildete 4-Hydroxy-2-oxo-pyrrolidin-1-yl-essigsäure verestert und der resultierende 4-Hydroxy-2-oxo-pyrrolidin-1-yl-essigsäureester auf bekannte Weise mit Ammoniak zum Endprodukt umgesetzt.

## Beschreibung

Die vorliegende Erfindung betrifft 4-(C₁-C₄)-Alkoxy-3-pyrrolin-2-on-1-yl-essigsäuren der allgemeinen Formel 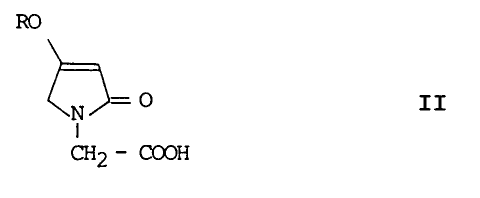 sowie deren Herstellung und Verwendung.

4-Hydroxy-2-oxo-pyrrolidin-1-yl-acetamid der Formel 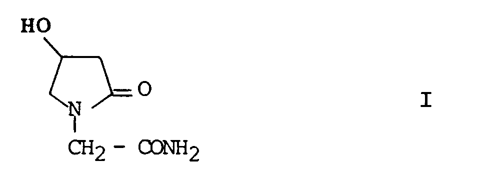 ist ein cerebral
wirksames Pharmazeutikum, das unter dem Namen Oxiracetam bekannt ist (Pifferi et al, Il Farmaco, Ed.Sc., 1977, 32, 602).

Es sind zahlreiche Synthesen bekannt, um den genannten Wirkstoff herzustellen.

So ist aus der EP-Anm. 224 256 bekannt, einen 4-Halogen-3-(C₁-C₂)-Alkoxy-2E-buten-säure-C₁-C₄-alkylester mit einem Glycinester in den entsprechenden 4-(C₁-C₂)-Alkoxy-3-pyrrolin-2-on-1-yl-essigsäurealkylester umzusetzen.

Aus der CH-PS 668 066 ist bekannt, diesen Ester mit Hilfe eines Palladiumkatalysators mit Wasserstoff zu hydrieren, danach die Alkoxygruppen mit Trichlormethylsilan und Natriumjodid zu entfernen und den resultierenden 4-Hydroxy-2-oxo-pyrrolidin-essigsäureester mit Ammoniak zum Endprodukt umzusetzen.

Aus der EP-Anm. 216 325 ist ausserdem bekannt, bei einem 4-(C₁-C₂)-Alkoxy-3-pyrrolin-2-on-1-yl-essigsäurealkylester die Alkoxygruppe zu hydrolysieren, den resultierenden 2,4-Dioxo-pyrrolidin-1-yl-essigsäurealkylester mit Natriumborhydrid zu hydrieren und den gebildeten 4-Hydroxy-2-oxo-pyrrolidin-1-yl-essigsäureester schliesslich mit Ammoniak zum Endprodukt umzusetzen.

Die bekannten Synthesen haben den Nachteil, dass insbesondere beim Hydrierungsschritt in erheblicher Menge unerwünschte 2-Oxo-pyrrolidin-1-yl-essigsäurederivate auftreten, die nur sehr schwierig und mit grossem Aufwand aus dem jeweiligen Reaktionsgemisch abgetrennt werden können.

Die gemäss EP-Anm. 224 256 bekannte Umsetzung mit Glycinester hat im weiteren aus oekologischer Sicht den Nachteil, dass zwingend in organischen Lösungsmitteln gearbeitet werden muss, um ein befriedigendes Resultat zu erreichen.

Die Aufgabe der Erfindung bestand daher darin, geeignete Ausgangstoffe für eine Synthese des 4-Hydroxy-2-oxo-pyrrolidin-1-yl-acetamids bereitzustellen, die die genannten Nachteile nicht aufweist und geeignet ist, in den technischen Maßstab umgesetzt zu werden.

Es wurde nun überraschend gefunden, daß die bisher unbekannten 4-(C₁-C₄)-Alkoxy-3-pyrrolin-2-on-1-yl-essigsäuren der allgemeinen Formel II hervorragend als Ausgangsstoffe für eine solche Synthese geeignet sind.

Gegenstand der vorliegenden Erfindung sind somit 4-(C₁-C₄)-Alkoxy-3-pyrrolin-2-on-1-yl-essigsäuren der allgemeinen Formel 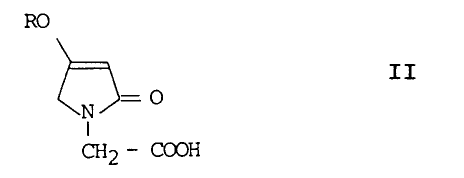 worin R (C₁-C₄)-Niederalkyl, vorzugsweise Methyl oder Ethyl, bedeutet.

Ein weiterer Gegenstand der vorliegenden Erfindung ist ein Verfahren zur Herstellung dieser 4-(C₁-C₄)-Alkoxy-3-pyrrolin-2-on-1-yl-essigsäuren.

Hierzu wird ein 4-Halogen-3-(C₁-C₄)-alkoxy-2E-butensäure-(C₁-C₄)-alkylester, vorzugsweise der 4-Chlor-3-methoxy-2E-butensäure-methylester, mit Glycin zur entsprechenden 4-(C₁-C₄)-Alkoxy-3-pyrrolin-2-on-1-yl-essigsäure umgesetzt.

Die Umsetzung mit Glycin wird zweckmässig in Wasser als Lösungsmittel in Gegenwart einer Base so durchgeführt, dass sich der pH des Reaktionsgemisches während der Reaktion zwischen 7 und 13 bewegt.

Geeignete Basen sind Alkali- oder Erdalkalihydroxide, -carbonate oder -hydrogencarbonate. Vorzugsweise wird Natriumhydroxid verwendet. Die Reaktionstemperatur bewegt sich zweckmäßig zwischen 50 und 100°C.

Die 4-(C₁-C₄)-Alkoxy-3-pyrrolin-2-on-1-yl-essigsäuren können auf übliche Weise aus dem Reaktionsgemisch isoliert werden. Gegebenenfalls können sie aber auch ohne vorherige Isolierung für weitere Umsetzungen eingesetzt werden, wie z.B. für die Herstellung des 4-Hydroxy-2-oxo-pyrrolidin-1-yl-acetamids.

Ein weiterer Gegenstand ist dementsprechend die Verwendung der 4-(C₁-C₄)-Alkoxy-3-pyrrolin-2-on-1-yl-essigsäuren zur Herstellung des 4-Hydroxy-2-oxo-pyrrolidin-1-yl-acetamids.

Hierzu wird die Alkoxygruppe sauer hydrolysiert. Zweckmässig wird diese Hydrolyse in wässrigen Mineralsäuren, wie wässriger Salzsäure, Bromwasserstoffsäure, Schwefelsäure, Salpetersäure oder Phosphorsäure, vorzugsweise in wässriger Salzsäure, bei Temperaturen zwischen 0 und 100°C durchgeführt.

Die resultierende 2,4-Dioxo-pyrrolidin-1-yl-essigsäure wird üblicherweise nicht isoliert, sondern direkt der Hydrierung zugeführt.

Die Hydrierung erfolgt mit Wasserstoff in Gegenwart eines Hydrierkatalysators.

Als Hydrierkatalysator können die üblichen Katalysatorsysteme, wie z.B. Platin, Ruthenium oder Rhodium, auf geeigneten inerten Trägern, wie z.B. Kohle, Aluminiumoxid oder Kieselgel, verwendet werden.
Besonders vorteilhafte Ergebnisse konnten mit einem Rutheniumkatalysator, aufgebracht in einer Menge von 1 bis 10% auf Kohle als Trägermaterial, erreicht werden.

Zweckmässig arbeitet man in einem wässrigen sauren Medium, bevorzugt im bei der vorgängigen Hydrolyse verwendeten Medium, in einem pH-Bereich zwischen 0 und 2, bei Temperaturen zwischen 0 und 70°C, und bei Wasserstoffdrucken zwischen 5 und 50 bar.

Die gebildete 4-Hydroxy-2-oxo-pyrrolidin-1-yl-essigsäure kann auf übliche Weise isoliert werden. Vorzugsweise wird sie aber direkt dem Veresterungsschritt zugeführt.

Die Veresterung erfolgt zweckmässig mit niederen aliphatischen Alkoholen mit bis zu 4 C-Atomen, wie Methanol, Ethanol, Propanol und seine Isomere oder Butanol und seine Isomere. Vorzugsweise wird Ethanol eingesetzt.
Man kann entweder in Gegenwart einer Mineralsäure, wie z.B. Schwefelsäure, oder in Gegenwart von Thionylchlorid die Veresterung durchführen.
Wird in Gegenwart einer Mineralsäure verestert, setzt man vorteilhaft zusätzlich ein geeignetes Lösungsmittel, wie z.B. Cyclohexan, Toluol, Benzol oder Hexan, zu, um das Reaktionswasser azeotrop aus dem Reaktionsgemisch zu entfernen.
Die Veresterung wird zweckmässig bei Temperaturen zwischen 20°C und der Rückflusstemperatur des betreffenden Alkohols, vorzugsweise bei Rückflusstemperatur des Alkohols, durchgeführt.
Vorzugsweise wird die Veresterung in Gegenwart von Thionylchlorid durchgeführt.

Der resultierende 4-Hydroxy-2-oxo-pyrrolidin-1-yl-essigsäure-(C₁-C₄)-alkylester kann nach üblicher Aufarbeitung der Umsetzung mit Ammoniak zum Endprodukt zugeführt werden.

Diese letzte Stufe, d.h. die Umsetzung mit Ammoniak, erfolgt auf bekannte Weise, z.B. gemäß EP-A-224 256.

Das 4-Hydroxy-2-oxo-pyrrolidin-1-yl-acetamid kann in guten overall-Ausbeuten und in sehr hoher Reinheit gewonnen werden.

### Beispiel 1

### Herstellung von 4-Methoxy-3-pyrrolin-2-on-1-yl-essigsäure

30 g (400 mmol) Glycin wurden in 60 ml H₂O aufgeschlämmt und auf 70°C erhitzt. Man gab mit einem Autotitrator 10M NaOH zu, bis der pH 8,5 war. Dann gab man bei 70-75°C 22,4 g (136 mmol) 4-Chlor-3-methoxy-but-2E-en-säuremethylester zu. Der pH-Wert wurde durch Zugabe von 10M NaOH auf 8,5 konstant gehalten. Nach 1 h gab man weitere 22,4 g (136 mmol) 4-Chlor-3-methoxy-but-2E-en-säuremethylester zu, und nach einer weiteren h nochmals 22,4 g (136 mmol)4-Chlor-3-methoxy-but-2E-en-säuremethylester.
Nach der letzten Zugabe rührte man noch 3,5 h bei 70-75°C weiter. Der Verbrauch an 10M NaOH betrug dann 80,3 ml, was 2 Aequivalenten bezüglich eingesetztem Glycin entsprach.
Man kühlte auf Raumtemperatur ab und verdünnte mit 40 ml H₂O. Danach stellte man mit konz. HCl auf pH 1 ein. Das Produkt fiel nach kurzer Zeit aus, wurde abfiltriert und mit gesättigter NaCl-Lösung gewaschen. Nach Trocknung erhielt man 70,9 g Produkt.

Aus der Mutterlauge wurden weitere 3,41 g Produkt gewonnen.
Gesamtausbeute: 84%.
Dieses Rohprodukt konnte aus Isopropanol umkristallisiert werden:
68 g wurden aus 450 ml Isopropanol umkristallisiert. Man erhielt 47 g Produkt, was 87% Ausbeute entsprach. Das Produkt enthielt weniger als 0,01% Cl⁻.
Smp. 164,0-164,5°C
- ¹H-NMR: (D⁶-DMSO): 3,78 (s, 3H); 3,98 (s, 2H)
4,02 (s, 2H); 12,75 (br.s, 1H)

### Beispiel 2

### Herstellung von 4-Hydroxy-pyrrolidin-2-on-1-yl-essigsäure

2,0 g (11,7 mmol) 4-Methoxy-3-pyrrolin-2-on-1-yl-essigsäure wurden in 15 ml HCl (1N) bei Raumtemperatur gelöst und unter Rühren auf 70°C erhitzt. Nach 1 h wurden 2,04 g Natriumacetat zugegeben, um den pH auf 2,0 zu stellen. Dazu gab man 100 mg Ru/C. Die Reaktionsmischung wurde in einen Autoklav transferiert und bei 10 bar H₂ Druck unter Rühren hydriert. Nach 18 h wurde der Katalysator abgenutscht und das Filtrat am Vakuum eingeengt.
Dies ergab 2,9 g einer Rohmischung von NaCl und 4-Hydroxy-pyrrolidin-2-on-1-yl-essigsäure (42% Gehalt, 60% Ausbeute).

### Beispiel 3

### Herstellung von 4-Hydroxy-pyrrolidin-2-on-1-yl-essigsäure-ethylester

Eine Reaktionsmischung, bestehend aus 3,05 g (19,3 mmol) 4-Hydroxy-pyrrolidin-2-on-1-yl-essigsäure, 20 ml Ethanol, 0,5 ml H₂SO₄ und 100 ml Cyclohexan, wurde während 4 h am Rückfluss gekocht. Das Reaktionswasser wurde im Wasserabscheider abgetrennt.
Man engte die Reaktionsmischung vollständig ein. Danach löste man den Rückstand in 35 ml 10% NaCl in H₂O, neutralisierte mit NaOH und extrahierte kontinuierlich mit Tetrahydrofuran während 18 h.
Die organische Phase wurde über MgSO₄ getrocknet und am Rotationsverdampfer eingeengt.
Nach Trocknung am Hochvakuum erhielt man 2,83 g Produkt.

### Beispiel 4

### Herstellung von 4-Hydroxy-pyrrolidin-2-on-1-yl-acetamid (Oxiracetam)

3,5 g (18,8 mmol) 4-Hydroxy-pyrrolidin-2-on-1-yl-essigsäure-ethylester wurden in 35 ml Ethanol gelöst und mit 14 g flüssigem Ammoniak versetzt. Dieses Gemisch wurde im Autoklaven bei 50°C während 16 h gerührt (Druck 6 bar). Man kühlte auf Raumtemperatur und entfernte das überschüssige Ammoniak. Die erhaltene Suspension wurde am Rotationsverdampfer eingeengt und am Hochvakuum getrocknet.
Man erhielt 2,95 g Oxiracetam (99%).
HPLC-Gehalt 97,1%
Smp. 165-167°C.

## Patentansprüche

1. 4-(C₁-C₄)-Alkoxy-3-pyrrolin-2-on-1-yl-essigsäuren der allgemeinen Formel 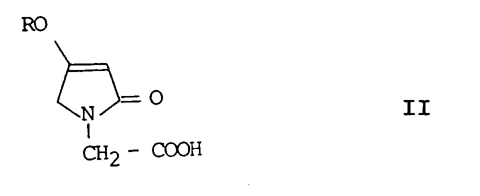 worin R (C₁-C₄)-Niederalkyl, vorzugsweise Methyl oder Ethyl, bedeutet.

2. Verfahren zur Herstellung von 4-(C₁-C₄)-Alkoxy-3-pyrrolin-2-on-1-yl-essigsäuren nach Patentanspruch 1, dadurch gekennzeichnet, daß man einen 4-Halogen-3-(C₁-C₄)-alkoxy-2E-butensäure-(C₁-C₄)-alkylester mit Glycin zum Endprodukt umsetzt.

3. Verfahren nach Patentanspruch 2, dadurch gekennzeichnet, daß die Umsetzung mit Glycin in Wasser und in Gegenwart einer Base bei einem pH-Wert zwischen 7 und 13 durchgeführt wird.

4. Verfahren nach mindestens einem der Patentansprüche 2 bis 3, dadurch gekennzeichnet, daß die Umsetzung mit Glycin bei Temperaturen zwischen 50 und 100°C durchgeführt wird.

5. Verwendung der 4-(C₁-C₄)-Alkoxy-3-pyrrolin-2-on-1-yl-essigsäuren nach Patentanspruch 1 zur Herstellung des 4-Hydroxy-2-oxo-pyrrolidin-1-yl-acetamids der Formel I 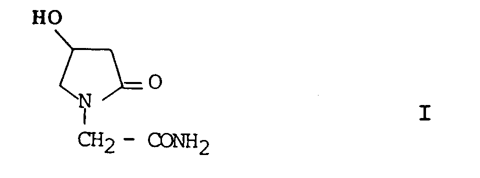 dadurch gekennzeichnet, daß man die Alkoxygruppen der 4-(C₁-C₄)-Alkoxy-3-pyrrolin-2-on-1-yl-essigsäure sauer hydrolyziert, die resultierende 2,4-Dioxopyrrolidin-1-yl-essigsäure in Gegenwart eines Hydrierkatalysators mit Wasserstoff hydriert, die gebildete 4-Hydroxy-2-oxo-pyrrolidin-1-yl-essigsäure verestert und den resultierenden 4-Hydroxy-2-oxo-pyrrolidin-1-yl-essigsäureester auf bekannte Weise mit Ammoniak zum Endprodukt umsetzt.

6. Verwendung nach Patentanspruch 5, dadurch gekennzeichnet, daß die saure Hydrolyse in wäßriger Mineralsäure bei Temperaturen zwischen 0 und 100°C durchgeführt wird.

7. Verwendung nach mindestens einem der Patentansprüche 5 bis 6, dadurch gekennzeichnet, daß die 2,4-Dioxopyrrolidin-1-yl-essigsäure nicht aus dem Reaktionsgemisch isoliert wird.

8. Verwendung nach mindestens einem der Patentansprüche 5 bis 7, dadurch gekennzeichnet, daß die Hydrierung mit einem Rutheniumkatalysator, aufgebracht auf einem inerten Träger, in saurem wäßrigen Medium, vorzugsweise bei einem Druck zwischen 5 und 50 bar und bei Temperaturen zwischen 0 und 70°C, durchgeführt wird.

9. Verwendung nach mindestens einem der Patentansprüche 5 bis 8, dadurch gekennzeichnet, daß die Veresterung mit aliphatischen Alkoholen mit bis zu 4 C-Atomen, entweder in Gegenwart einer Mineralsäure oder mit Thionylchlorid, bei Temperaturen zwischen 20°C und Rückflußtemperatur des betreffenden Alkohols erfolgt.
